# EUROPEAN PATENT APPLICATION

(11) **EP 3 321 899 A1**
(43) Date of publication of application: **16.05.2018**
(21) Application number: 16198142.8
(22) Date of filing: 10.11.2016
(51) Int. Cl.: G08B 6/00, G08B 21/24, A61J 7/04, A61K 9/70

(54) **ELECTRONIC COMMUNICATION UNIT FOR AN ADHESIVE PATCH**

(71) Applicant: Therasolve NV, 3590 Diepenbeek (BE)
(72) Inventor: Jans, Stijn Juliaan Bert, 3590 Diepenbeek (BE); Hermans, Ruben Felix Eleonora, 3590 Diepenbeek (BE); Janssens, Johan, 3590 Diepenbeek (BE)
(74) Representative: V.O.

(57) **Abstract**

An electronic communication unit for an adhesive patch arranged to be placed on a person's skin, said adhesive patch comprising a disposable patch component and a reusable patch component, wherein a bottom surface of said disposable patch component comprises an adhesive layer for detachably securing said adhesive patch on said person's skin, wherein said reusable patch component comprises said electronic communication unit.

## Description

The invention relates to an electronic communication unit for an adhesive patch arranged to be placed on a person's skin.

Poor adherence and nonadherence to medication by patients has been recognised as an important health issue, as it is a key factor that is associated with the effectiveness of pharmacological therapies, particularly in case of chronic conditions such as diabetes, heart disease, respiratory disease and cancer. It is generally believed that poor or non-adherence to medication is directly related to poor clinical outcomes, high health care costs and lost quality of life. The cost of medication non-adherence has been estimated to mount up to hundreds of billions annually, including costs from hospitalization, nursing home admissions and premature deaths, which could have been avoided. Nevertheless, long-term adherence to medication for treating those diseases has been found to be poor. For example, despite the fact that pharmacological antihypertensive therapy reduces the risk of stroke and myocardial infarction, there is evidence that the majority of the patients treated for hypertension are non-adherent to their treatment regimen. Other representative examples include anti-diabetes and lipid-lowering therapies, which were found to significantly reduce the risk of ischemic events. Non-adherence to medicines has been observed to take many forms from simply forgetting to take a prescribed medicine on the necessary fixed points of time and substituting this by a later point of time, to forgetting to take a prescribed medicine at all, being the most common forms.

It is generally believed that with a consistent use of the medication as prescribed, the chances to a successful treatment of a disease can be increased and that many of the medical costs associated with medication nonadherence can be avoided. Closing the adherence gap is therefore considered important to the success of initiatives to improve the quality of health care, and is thought to have an impact that may be of similar importance or even better than any improvement to specific medical treatments.

To assist a patient in improving his medication adherence, electric communication units have been developed. In a practical example, an electric communication unit can take the form of a wearable device, for example a patch to be placed on a person's skin.

Aided by the increased processing speed of chips, capacity of batteries and precision of sensor technologies, nowadays wearable devices are becoming smaller, faster and more feature heavy, nearly replacing entire electronic categories in the process and furthermore impacting the way we live, work and socialize. Unlike traditional consumer electronics, wearable devices are in physical contact with their users up to 24 hours a day or sometimes for considerably longer periods of time, for example whenever they are equipped with bio-sensors such as CGM (Continuous Glucose Monitoring) sensors. Such wearable devices interface with the body and therefore need to be designed for long term skin contact. In case they are equipped with bio-sensors they need to also ensure the reliability of that skin contact under a variety of real life circumstances. Whilst adhesive patches are often called upon to provide the required level of reliability of skin and /or subdermal contact, they pose an additional challenge: i.e. they need to be replaced every so often as the adhesive loses its function over time. Each replacement of the adhesive patch involves loss of the sometimes costly wearable device and/or sensor. Finding techniques which would permit the wearable device and/or sensor to be recovered from the adhesive patch for future re-use, ease of removal and reassembly from the adhesive patch as well as ensuring the reliability of the contact with the skin and/or subdermal skin layers are just a few of the challenges to be met in case a wearable device and/or sensor is to make contact with the skin by means of an adhesive patch.

WO2005/119610 discloses an electric communication unit, which comprises an ASIC (Application Specific Integrated Circuit), which is positioned on a support element and connected with a power supply, a receiver-antenna and body contacts. A cover consisting of a thin flexible synthetic material is used to protect all fragile electronic components against external influences. A pulse generator is provided for generating and transmitting a series of pulses to the body contacts in the patch upon receipt of a first signal. The body contacts transmit the series of pulses onto the skin of said person. The pulse generator further comprises a processing unit for storing a second signal, which comprises data indicative at which time said first signal should be generated, and for generating and transmitting the first signal to the pulse generator. An electric communication unit of this kind is capable of improving medication adherence because the user is alerted by the pulses on the skin that he or she has to take his or her medicine, or has to administer a medicine to a patient. The adhesive patch with electric communication unit disclosed in WO2005/119610 can be applied on the skin of the patient or on the skin of another person taking care of a patient. It is thus not necessary for the patient to wear the electric communication unit. An adhesive on the bottom side of the support element permits to position and stick the patch on the skin. A liner protects the adhesive of a not-yet used patch. The electric communication unit may further comprise an area on which a drug is adsorbed, in which case the electric communication unit may be used as a transdermal patch.

However, because the patch is adhered to the skin, it will be subjected to the conditions encountered by the skin, e.g. it will be contacted with water and detergent, for example during showering. It will be subjected to movement and to mechanical forces exerted by the clothing. These conditions have been found to limit the lifetime of the adhesive to several days, the number of which may vary between different users, and generally lasts for about a maximum of about 5-10 days, often about a week. Although the electronics part and the power source contained therein may have a much longer lifetime, the patch will need replacement because the adhesive strength is becoming insufficient. When analyzing the disadvantages associated with the known patch, the inventors observed that the patch will usually need to be exchanged because the carrier does no longer adhere to the skin, substantially before the lifetime of the electronics part and the power source has been exceeded. Therefore, the cost of the electronics part and power source determine the cost of the patch.

The inventors have therefore sought for a solution to increase the lifetime of the patch, thus decreasing the cost of the patch and making it available in this way for use by a broader public.

WO2015/048298 discloses a remote physiological sensing device, comprising a holder, first and second electrodes coupled with the holder and an electronics package removably coupled with the holder and in electrical contact with the first and second electrodes via the holder. The electronics package includes a housing, a wireless transceiver and electronic circuitry configured to process signals received via the first and second electrodes and the wireless transceiver. The electronics package may be personalized for a use by a user, and may be removed from the disposable holder and then reinserted into a replacement holder or patch. The sensing unit may be coupled to a patient's chest or any other suitable portion of the patient's body, such as the patient's arm or thigh, using an adhesive or any other suitable mechanism, such as a strap. Alternatively, the sensing unit may be coupled to or integral with a garment configured to be worn by the patient, for example, a belt, a wristband, a headband, an armband, shorts, pants, and/or the like. The first and second electrodes may be integrally formed with the holder or patch and may be disposable therewith. The holder or patch may include a battery.

In the device disclosed in WO2015/048298, the holder has the function of housing the battery and ensuring electric contact between the electrodes and the electronics package. Skin contact of the device is ensured by the electrodes. After replacement of the electronics package into a disposable holder, contact between the electronics package and the skin of the wearer via the holder may not be optimal. This is, however, not critical for the disclosed device, as it has not been configured to provide tactile signals to the skin. Only the electrodes need to make contact with the skin. In addition to this drawback, reassembly of the reusable parts with the disposable parts may be rather complicated.

It is an aim of the present invention to solve or alleviate one or more of the above-mentioned problems. The present invention seeks to provide a solution to prolong the use time of at least the electronics part contained in the patch and maximizing ease of replacement of a disposable part of the patch, especially for the chronically ill, without adversely affecting the reliability of the timing of the signals generated by the electronics part. In particular, it is an aim of the invention to provide an electronic communication unit for an adhesive patch, which is able to timely transmit tactile signals to a user in spite of exchange of a disposable part of the patch.

This is achieved according to a first aspect of the present invention by an electric communication unit, which shows the technical features of the characterizing portion of the first claim. In particular, the present invention provides an electronic communication unit for an adhesive patch. The adhesive patch is arranged to be placed on a person's skin, and comprises a disposable patch component and a reusable patch component, wherein a bottom surface of said disposable patch component comprises an adhesive layer for detachably securing said adhesive patch on said person's skin. The reusable patch component comprises the electronic communication unit, wherein said electronic communication unit comprises a lifetime counter arranged to keep a tally of a counter value counting a time from a lifetime starting point of said electronic communication unit, wherein said electronic communication unit comprises a tactile signal generator arranged to generate and transmit at least one tactile signal to said person's skin on at least one preprogrammed point in time, wherein said at least one preprogrammed point in time is based on said electronic communication unit's lifetime counter, wherein said lifetime counter is arranged to continue counting irrespective of an exchange of said disposable patch component with a next disposable patch component. In this way, transmission of at least one tactile signal to a person's skin can occur on at least one pre-programmed point in time, which can be conveniently chosen and programmed, and which can be maintained in a reliable manner, independently of an exchange of the disposable patch part for another disposable patch part. As the lifetime counter does not stop counting during an exchange of the disposable patch part, the transmission of the at least one tactile signal to a person's skin will not be shifted in time because of the exchange.

In a preferred embodiment of the invention, said at least one tactile signal can be a replacement signal indicating need for exchange of said disposable patch on said at least one preprogrammed point in time, or can be an administration signal indicating need for administration of a medicine on said at least one point in time. The tactile signal will be felt by the wearer of the patch, and may be associated to an action to be performed by the user of the patch. The tactile signal may always be a similar signal or may be a different signal, for example in duration and/or strength, according to the action that is required. Such an action may for example include the administration of a medicine, by the user or to another patient not wearing the patch, for example to a child or to an elderly person. The action may also comprise the exchange of the disposable patch part. The disposable patch part may for example need to be exchanged because the adhesive layer can only guarantee sufficient adhesion during a limited period of time, for example during 3 to 7 days. Additionally or alternatively, the disposable patch part may for example include a medicine configured to be administrated transdermally through said person's skin. In that case, the disposable patch part may need to be replaced when there is no medicine contained in the disposable patch part anymore. The tactile signal might also be combined with an audible and/or visual signal, which may however have less or no effect in case the user of the patch is sleeping, or to the contrary, is in a noisy environment.

Said electronic communication unit can preferably be configured to function in a use mode, in which mode said a tactile signal generator is arranged to generate and transmit at least one tactile signal to said person's skin, and in a power saving mode, in which mode said tactile signal generator is arranged not to generate or transmit any signal, wherein said power saving mode is activatable after at least a predetermined time, wherein said predetermined time is preferably substantially shorter than an estimated lifetime of a power source of said electronic communication unit. This feature can enhance the reliability of the device, as it can prevent an interruption of signals due to a lack of power source.

In a more preferred embodiment, said lifetime counter is arranged to be stopped just before activation of said power saving mode. As the life time counter is arranged to count the lifetime during which the electronic communication unit can be actively used, i.e. generating and transmitting tactile signals to a person's skin, there is no reason to let the lifetime counter continue counting, so power can be saved by stopping the lifetime counter.

It is still more preferred that the electronic communication device comprises a warning element arranged to provide a warning signal when said electronic communication unit switches to said power saving mode. Such a warning element can warn the user that the reusable patch part will be switched into the power saving mode, and that as a consequence, the user will not receive any signal anymore. The warning signal can for example be a tactile signal, similar to or different from the replacement signal and/or the administration signal, or can also be a visual or audible warning signal, or a combination thereof.

In a very advantageous embodiment, said predetermined time, after which said power saving mode is activatable, may be a multiple of a recommended time of use of said disposable patch component. As an example, it may be recommended to use a disposable patch part not more than three consecutive days. In that case, the activation of the power saving mode may be pre-programmed for example 21 days after the lifetime starting point, as long as the estimated lifetime of the power source is substantially more than 21 days. By doing so, the use of the disposable patch part is optimized, avoiding a switch to the power saving mode, when a disposable patch part may still be of use.

In a more preferred embodiment, the electronic communication unit can comprise at least one sensing device arranged to detect contact of said electronic communication unit with said person's skin, wherein said lifetime starting point corresponds to a first positive detection of contact of said electronic communication unit with said person's skin, wherein said signal generator is preferably arranged to generate and transmit a recognition signal to a person's skin confirming said first positive detection of contact of said electronic communication unit with said person's skin. When a person applies the adhesive patch to his skin for the first time, the sensing device can detect contact and reassure the user by signaling the recognition of contact. From that point on, the lifetime counter can be started. Alternatively, in absence of said sensing device, the lifetime counter could also be started manually when the adhesive patch is applied for the first time, for example by connecting the electronic communication unit to a power source.

Said signal generator can preferably also be arranged to generate and transmit a recognition signal to a person's skin confirming a first positive detection of contact of said electronic communication unit with said person's skin after at least one detection of loss of contact. This can for example be the case after an exchange of the disposable patch part with a next disposable patch part. The signal confirming skin contact can reassure the user that the device is well positioned on the skin so that the at least one tactile signal that will be generated can be perceived well by the user.

It may be advantageous when the sensing device is arranged to check contact of said electronic communication unit with said person's skin at regular time intervals, for example every minute, preferably every 30 seconds. A positive detection of contact of said electronic communication unit with said person's skin can for example include an uninterrupted series of a predetermined number of skin contact detections by said at least one sensing device, for example a series of five consecutive skin contact detections. A detection of loss of contact of said electronic communication unit with said person's skin can for example include an uninterrupted series of a predetermined number of failures of skin contact detections by said at least one sensing device, for example a series of two consecutive failures of skin contact detections. In this way, the chance of false detections, hence warnings, can be decreased. As good skin contact is essential for tactile signals to be transmitted, it is preferred to have a relatively long series of skin contact detections before a positive detection is confirmed. In the same sense, loss of contact should be signaled as soon as possible, for example after only two failures of skin contact detection.

Said electronic communication unit can preferably be configured to function in a replacement mode, in which mode exchange of said disposable patch part is possible, after a predetermined time, for example after a recommended time of use of said disposable patch component, and/or when said sensing device detects a loss of contact of said electronic communication unit with said person's skin. In the replacement mode, the lifetime counter continues counting in spite of a possible exchange of the disposable patch part. The replacement mode can be initiated by two possible scenarios. In a first example, the user wears the adhesive patch as prescribed, and the replacement mode will be initiated automatically, i.e. after a pre-determined time, preferably after a recommended time of use of said disposable patch component, of for example 3 to 7 days, and a replacement signal will be generated. In another scenario, the user of the patch may remove the patch too early, i.e. before the recommended time of use, and before having received a replacement signal, intentionally, for example because of irritation, or unintentionally. In that case, the sensing device will detect loss of skin contact, for example after two consecutive failures of skin contact detection, and switch to a replacement mode.

In a more preferred embodiment, a power saving mode, in which mode said tactile signal generator is arranged not to generate or transmit any signal, is configured to be activated after at least a predetermined time in said replacement mode. The power saving mode can for example be activated after at least a predetermined time in said replacement mode in combination with a detection of loss of contact of the electronic communication device with the person's skin. Alternatively, the power saving mode can also be activated after a predetermined time in said replacement mode, without any further dependence on skin contact. From the moment the replacement mode is activated, or from the moment loss of skin contact is detected, the electronic communication unit can comprise another counter arranged to keep a tally of a counter value counting a time from the beginning of said replacement mode, or from the moment of detection of loss of skin contact. Once said replacement mode counter exceeds a predetermined value, for example of one hour, the electronic communication unit can be switched to the power saving mode. So, when the user takes too much time to exchange the disposable patch part for another one, or takes off the patch for too long, the patch can no longer be used, thus preventing that the user might miss a signal during a too long interruption in the use of the patch, or in case of a patch containing a transdermal medicine, preventing that the treatment loses its effect because of a too long interruption in the use of the patch.

In an advantageous embodiment, said signal generator can be arranged to repeat transmission of a tactile replacement signal indicating need for exchange of said disposable patch on said at least one preprogrammed point in time after transmission of a first tactile replacement signal to said person's skin when said sensing device detects contact of said electronic communication unit with said person's skin. After transmission of a first tactile replacement signal to said person's skin, when the electronic communication unit enters a replacement mode, the user may not react immediately, for example when the user is busy. As long as the sensing device detects skin contact, the tactile replacement signals may be repeated at regular time intervals, for example every 5 - 15 minutes, during a predetermined time interval or a predetermined number of times, for example 10 - 20 repetitions of a replacement signal, inciting the user to exchange the disposable patch part, which may loose its functionality.

In a more preferred embodiment, the electronic communication unit can further comprise an effective wear time counter arranged to keep a tally of a counter value counting a time during which contact of said electronic communication unit with said person's skin is detected, wherein said effective wear time counter is arranged to be started at a first positive detection of contact of said electronic communication unit with said person's skin. The effective wear time counter can allow a caretaker to check a patient's therapy adherence, or at least check during how much time the patient has effectively used the adhesive patch.

It is still more preferred when said effective wear time counter can be arranged to be interrupted after a preprogrammed time, for example after a recommended time of use of said disposable patch component, and/or after a detection of loss of contact of said electronic communication unit with said person's skin. In the first case, the wear time is able to count only effective time on the skin, for example not counting time exceeding the recommended time of use, during which the disposable patch part might not adhere well to the skin anymore, or not provide any transdermal medicine anymore. In case of a, (un)intentional patch removal before the recommended time of use has expired, the wear time counter can be arranged to be interrupted as well, again such that only effective time on the skin of the disposable patch part is counted. The combination of both features can give a relatively realistic idea of treatment adherence of the patient.

In an advantageous embodiment, the electronic communication unit can comprise a readable memory arranged to store results of said lifetime counter and/or of said effective wear time counter, wherein said memory is readable during said power saving mode, preferably only during said power saving mode. As the electronic communication unit can be arranged such that it does not give any signals to the skin anymore during said power saving mode, the wearer of the patch can take off the patch and bring it to a caretaker, for example a doctor, or pharmacist, to be read out. The memory can be arranged to be read out in an easy way for example by a dedicated reader device, for example by via wired contact with electrodes from the sensing device, such that the reading out requires a minimum of power from the electronic communication unit. Many other alternatives are possible here and are known to the person skilled in the art.

A second aspect of the invention can provide an adhesive patch arranged to be placed on a person's skin, said adhesive patch comprising a disposable patch component and a reusable patch component, wherein a bottom surface of said disposable patch component comprises an adhesive layer for detachably securing said adhesive patch on said person's skin, wherein said reusable patch component comprises an electronic communication unit according to the first aspect of the invention. Said patch can provide one of the above-mentioned advantages.

The present invention will be further elucidated with reference to exemplary embodiments. Corresponding elements are designated with corresponding reference signs.
Figure 1 shows a schematic timeline of use of a first preferred embodiment of an electronic communication unit according to the invention;
Figure 2 shows a schematic timeline of use of a second preferred embodiment of an electronic communication unit according to the invention.

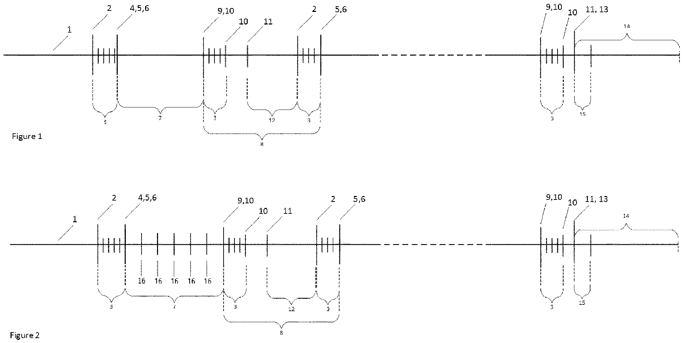

Figure 1 shows a schematic timeline of use of a first preferred embodiment of an electronic communication unit according to the invention. The electronic communication unit for an adhesive patch comprising a disposable patch component and a reusable patch component can for example be handed out to a user by a caretaker, a pharmacist and/or a doctor, who can pre-program the electronic communication unit comprised in the reusable patch component before its first use. From the electronic communication unit's fabrication until the first use, a preferred embodiment of the electronic communication mode can for example function in a pre-use low power mode 1, in which for example only the sensing device may be active and the unit can be programmed. The user may for example need to wear an adhesive patch of which the disposable patch component contains a transdermal medicine. Due to the limited amount of medicine contained in the adhesive patch, the disposable patch component may for example need to be replaced every 3.5 days. The lifetime counter may be programmed such that the power saving mode of the electronic communication unit is activated after for example at least 21 days, which is a multiple of the recommended time of use (3.5 days) of the disposable patch component, and is substantially shorter than the estimated lifetime, of for example 30 days, of the power source of the electronic communication unit. The user can now decide to start wearing the adhesive patch at a first application point in time 2, preferably in the morning or the evening, for example at 06:56h. The sensing device can now start to check contact 3 of the electronic communication unit with the person's skin, preferably at regular time intervals, for example every minute, or every 30 seconds. After an uninterrupted series of for example four skin contact detections, the sensing device may conclude to a first positive detection of contact of the electronic communication unit with said person's skin, which may be considered as a lifetime starting point for the lifetime counter as well as for the wear time counter, of which counting may be activated at a second point in time 4, 5, for example at 7:00h. In order to reassure the user of the adhesive patch that skin contact has been detected, the tactile signal generator may be arranged to generate and transmit a recognition signal 6 to the person's skin confirming the first positive detection of contact of said electronic communication unit with the person's skin.

After 3.5 days, i.e. the recommended time of use 7 of the disposable patch component, the electronic communication unit is configured to function in a replacement mode 8, in which mode exchange of the disposable patch part is possible. The lifetime counter continues counting irrespective of an exchange of said disposable patch component with a next disposable patch component. The effective wear time counter is arranged to be interrupted at point 9 in time, for example at 19:00h, and the tactile signal generator will generate and transmit a first tactile signal, a replacement signal 10, to said person's skin user, indicating need for exchange of the disposable patch component. In case the sensing device continues to detect contact of said electronic communication unit with said person's skin, the signal generator may be arranged to repeat transmission of a tactile replacement signal 10 indicating need for exchange of said disposable patch after transmission of a first tactile replacement signal to said person's skin, for example every 15 minutes. The user can for example remove the adhesive patch at point 11, for example at19:29h after a second replacement signal at 19:15h. The user may for example decide to remain some time without patch during an interruption time 12, for example to let the skin calm down. The user can for example reapply the adhesive patch, comprising a next disposable patch component and the same electronic communication device in the reusable patch component, at a next application point in time 2, for example at 20:16h. As soon as the sensing device has registered a uninterrupted series of for example four skin contact detections 3, the replacement mode 8 can end. The effective wear time counter can be started again at point 5 in time, for example at 20:20h, and the tactile signal generator can be arranged to generate and transmit a recognition signal 6 to the person's skin confirming a first positive detection of contact of said electronic communication unit with the person's skin after at least one detection of loss of contact, i.e. during the replacement mode 7.

In case of correct use of the adhesive patch, the above-mentioned scenario will be repeated for example six times, so that six disposable patch components will be used for a single reusable patch component comprising the electronic communication unit. The replacement mode 7 will always be activated at the same moment of the day, for example alternatingly at 07:00h and 19:00h, because these preprogrammed points in time are based on said electronic communication unit's lifetime counter. After at least a predetermined period of time, in this example after 21 days, corresponding to the end of the sixth period of 3.5 days, the electronic communication unit is configured to function in a power saving mode. Just before the power saving mode is activated, the effective wear time counter will be stopped at time 9, for example at 19:00h, and the tactile signal generator will generate and transmit a first tactile signal again, a replacement signal 10, to said person's skin user, indicating need for exchange of the disposable patch component. Said replacement signal may again need to be repeated in case the sensing device continues to detect contact of said electronic communication unit with said person's skin. As soon as loss of skin contact is detected by the sensing device, because the user has for example removed the adhesive patch at time 11, for example at 19:20h after a second replacement signal 10 at 19:15h, the lifetime counter is stopped 13 and counter values of the effective wear time counter and the lifetime counter are stored on the readable memory. The power saving mode 14 is now activated, and a warning element, for example a blinking LED, may be activated during a period in time 15, for example one hour, to signal the switch of the electronic communication unit to the power saving mode, in which mode said tactile signal generator is arranged not to generate or transmit any signal anymore. The user can now return to the caretaker who handed out the adhesive patch, who can read out the electronic communication unit's memory with a dedicated device, in order to have an idea of the patient's therapy adherence.

It may happen that a user does not react to the tactile signals indicating need for exchange of said disposable patch component, in spite of a series of repetitions of said replacement signals. In case the user leaves the patch on the skin and the sensing device continues detecting skin contact, the electronic communication unit may remain in a replacement mode, i.e. with the effective wear time counter being stopped and the lifetime counter counting, for an extended period of time, for example until the signal generator transmits a replacement signal of a next period to the skin, or for example until the end of the last predetermined period of use, in this example after 21 days. When the electronic communication unit's memory is read out, the non-compliance of the patient will be detected because of the effective wear time counter value being substantially shorter than the life time counter value.

Another problematic behaviour of the user may consist in removing the adhesive patch after a replacement signal without timely replacing the disposable patch component with a next disposable patch component. The electronic communication unit may then be configured to be switched from the replacement mode into the power saving mode after a predetermined period of time during which no skin contact is detected, for example after one hour without positive skin contact detection. As described above, the lifetime counter will be stopped, the counter values of the lifetime counter and the effective wear time counter will be stored in the memory and a warning signal may be activated. Again, the non-compliance of the patient will be detected when the memory of the electronic communication unit is be read out.

In case the user removes the adhesive patch (un)intentionally without any replacement signal indicating to do so, the sensing device can detect a loss of contact of said electronic communication unit with the person's skin, and the electronic communication unit can be configured to switch to a replacement mode and to stop the effective wear time counter. As soon as the patch is applied to the skin, with the same disposable patch component or a new disposable patch component, and skin contact is again detected by the sensing device starting the effective wear time counter and switching to use mode again, the use of the patch can continue as before. However, even if the user has prematurely exchanged the disposable patch component for a new one, the next replacement signal will remain as scheduled originally.

Figure 2 shows a schematic timeline of use of a second preferred embodiment of an electronic communication unit according to the invention. In this second preferred embodiment of the invention the disposable patch component does not contain any medicine. The electronic communication unit for an adhesive patch comprising a disposable patch component and a reusable patch component can for example be worn by a patient needing to take a medicine at very regular time intervals, for example once or twice a day at fixed points, for instance at 7:00h in the morning. The adhesive patch can in this embodiment also be worn by a caretaker, for example a caretaker and/or nurse of children or elderly people who need to have a medicine administered regularly. The recommended time of use of the disposable patch component is now determined in particular by the adhesive layer of the disposable patch component, which can for example guarantee a good adherence to the skin during seven days. Again, it is advantageous to program the electronic communication unit for a start of the power saving mode after at least a multiple of said recommended time of use 7 of the disposable patch part while taking into account a substantial margin for the estimated lifetime of the unit's power source, so for example for a period of 21 days. Before its first use, the electronic communication unit can for example be programmed to generate and transmit at least one tactile signal to said person's skin every seven days after the lifetime starting point, if this is for example the recommended time of use 7 of the patch, said signal being a replacement signal indicating need for exchange of said disposable patch component. At the same time, the electronic communication unit can be programmed to generate and transmit at least one tactile signal to said person's skin at predetermined points in time, for example every morning at 7:00h, said signal being an administration signal indicating need for administration of a medicine on said at least one point in time. The replacement signal and the administration signal may preferably be different tactile signals, so as to distinguish the action the signal intends to remind the user of. In case of different medicines which need to be taken at different moments of the day, for example one medicine at 7:00h and another medicine at 13:00h, the administration signal may also be different for each of these two points in time, for example differing in length and/or intensity. The patch is preferably applied to the skin on, or just after, but not just before, a point in time on which a medicine needs to be taken. The functioning of the electronic communication unit is largely analogous to the embodiment of Figure 1 described extensively above: after a positive skin contact check 3, the lifetime counter and the effective wear time counter can be initiated at points 4 and 5, preferably accompanied by a skin contact recognition signal 6. During the recommended time of use 7 of the adhesive patch, the tactile signal generator can be programmed to generate and transmit at least one tactile signal to the person's skin on at least one preprogrammed point in time 16, for example every day at 07:00h, wherein said at least one preprogrammed point in time 16 is based on the electronic communication unit's lifetime counter.

After the recommended time of use 7 of the disposable patch component, the switch of the electronic communication unit into a replacement mode and the restarting of the wear time counter can function analogously to the first embodiment. Also the possible anomalies, as well as the switch to the power saving mode can be treated in an analogous way. The timing of the replacement mode 8, and more specifically of the interruption time 12 between the removal of the adhesive patch and the reapplication of the patch, can be slightly more critical, in that it may be important not to miss any administration signal. This can be partly avoided by strictly limiting the allowed replacement mode 8 or only the interruption time 12, for example to one hour, and by applying the adhesive patch for a first use within for example one hour before a time 16 on which a medicine needs to be taken.

Also the reading out of the memory can be processed in a same way as described for the first embodiment of Figure 1. However, the registered values of the wear time counter and the lifetime counter can only give an indication of a compliant use of the adhesive patch, not of the compliance to the medicine that needs to be taken. This embodiment of the adhesive patch can therefore more be considered as a useful help in reminding a caretaker and/or patient of having to take an/or administer a medicine, not so much as a control tool for caregivers of patient compliance.

In an attempt not to surcharge Figures 1 and 2, the contact checks with the user's skin at regular intervals are only indicated at certain periods, even if they can be, and preferably are, continuously repeated, for example every minute until the electronic communication unit switches to the power saving mode. In these figures, the use mode, starting at the start of the lifetime counter and running until the stopping of the lifetime counter, has not been indicated.

It should be clear to the person skilled in the art that the invention is not limited to the embodiments described above. Many alternatives are possible within the scope of protection as formulated in the claims hereafter.

### List of references

- 1: Pre-use low power mode
- 2: Application of adhesive patch
- 3: Skin contact checks
- 4.: Start of lifetime counter
- 5: Start of effective wear time counter
- 6: Skin contact recognition signal
- 7.: Recommended time of use
- 8: Replacement mode
- 9: Stop of effective wear time counter
- 10: Replacement signal
- 11: Removal of adhesive patch
- 12: Interruption time
- 13: Stop of lifetime counter
- 14: Power saving mode
- 15: Warning element activation time
- 16: Administration signal

## Claims

1. An electronic communication unit for an adhesive patch arranged to be placed on a person's skin, said adhesive patch comprising a disposable patch component and a reusable patch component, wherein a bottom surface of said disposable patch component comprises an adhesive layer for detachably securing said adhesive patch on said person's skin, wherein said reusable patch component comprises said electronic communication unit, wherein said electronic communication unit comprises a lifetime counter arranged to keep a tally of a counter value counting a time from a lifetime starting point of said electronic communication unit, wherein said electronic communication unit comprises a tactile signal generator arranged to generate and transmit at least one tactile signal to said person's skin on at least one preprogrammed point in time, wherein said at least one preprogrammed point in time is based on said electronic communication unit's lifetime counter, wherein said lifetime counter is arranged to continue counting irrespective of an exchange of said disposable patch component with a next disposable patch component.

2. An electronic communication unit according to claim 1, wherein said at least one tactile signal is a replacement signal indicating need for exchange of said disposable patch component on said at least one preprogrammed point in time, or is an administration signal indicating need for administration of a medicine on said at least one point in time.

3. An electronic communication unit according to any of the preceding claims, wherein said electronic communication unit is configured to function in a use mode, in which mode said a tactile signal generator is arranged to generate and transmit at least one tactile signal to said person's skin, and in a power saving mode, in which mode said tactile signal generator is arranged not to generate or transmit any signal, wherein said power saving mode is activatable after at least a predetermined time, wherein said predetermined time is preferably substantially shorter than an estimated lifetime of a power source of said electronic communication unit.

4. An electronic communication unit according to claim 3, wherein said lifetime counter is arranged to be stopped just before activation of said power saving mode.

5. An electronic communication unit according to any of the preceding claims 3-4, comprising a warning element arranged to provide a warning signal when said electronic communication unit switches to said power saving mode.

6. An electronic communication unit according to any of the preceding claims, comprising at least one sensing device arranged to detect contact of said electronic communication unit with said person's skin, wherein said lifetime starting point corresponds to a first positive detection of contact of said electronic communication unit with said person's skin, wherein said signal generator is preferably arranged to generate and transmit a recognition signal to a person's skin confirming said first positive detection of contact of said electronic communication unit with said person's skin.

7. An electronic communication unit according to claim 6, wherein said signal generator is arranged to generate and transmit a recognition signal to a person's skin confirming a first positive detection of contact of said electronic communication unit with said person's skin after at least one detection of loss of contact.

8. An electronic communication unit according to any of the preceding claims 6-7, wherein the sensing device is arranged to check contact of said electronic communication unit with said person's skin at regular time intervals, for example every minute, preferably every 30 seconds, wherein a positive detection of contact of said electronic communication unit with said person's skin includes an uninterrupted series of a predetermined number of skin contact detections by said at least one sensing device, and wherein a detection of loss of contact of said electronic communication unit with said person's skin includes an uninterrupted series of a predetermined number of failures of skin contact detections by said at least one sensing device.

9. An electronic communication unit according to any of the preceding claims 6-8, wherein said electronic communication unit is configured to function in a replacement mode, in which mode exchange of said disposable patch part is possible, after a predetermined time, for example after a recommended time of use of said disposable patch component, and/or when said sensing device detects a loss of contact of said electronic communication unit with said person's skin.

10. An electronic communication unit according to claim 9, wherein a power saving mode, in which mode said tactile signal generator is arranged not to generate or transmit any signal, is configured to be activated after at least a predetermined time in said replacement mode.

11. An electronic communication unit according to any of the preceding claim 6-10, wherein said signal generator is arranged to repeat transmission of a tactile replacement signal indicating need for exchange of said disposable patch on said at least one preprogrammed point in time after transmission of a first tactile replacement signal to said person's skin when said sensing device detects contact of said electronic communication unit with said person's skin.

12. An electronic communication unit according to any of the preceding claims 6-11, further comprising an effective wear time counter arranged to keep a tally of a counter value counting a time during which contact of said electronic communication unit with said person's skin is detected, wherein said effective wear time counter is arranged to be started at a first positive detection of contact of said electronic communication unit with said person's skin.

13. An electronic communication unit according to claim 12, wherein said effective wear time counter is arranged to be interrupted after a preprogrammed time, for example after a recommended time of use of said disposable patch component, and/or after a detection of loss of contact of said electronic communication unit with said person's skin.

14. An electronic communication unit according to any of the preceding claims 12-13, comprising a readable memory arranged to store results of said lifetime counter and/or of said effective wear time counter, wherein said memory is readable during said power saving mode, preferably only during said power saving mode.

15. Adhesive patch arranged to be placed on a person's skin, said adhesive patch comprising a disposable patch component and a reusable patch component, wherein a bottom surface of said disposable patch component comprises an adhesive layer for detachably securing said adhesive patch on said person's skin, wherein said reusable patch component comprises an electronic communication unit according to any of the preceding claims.
